# EUROPÄISCHE PATENTSCHRIFT

(11) **EP 1 094 111 B1**
(45) Veröffentlichungstag und Bekanntmachung des Hinweises auf die Patenterteilung: **30.08.2006**
(21) Anmeldenummer: 00121715.7
(22) Anmeldetag: 05.10.2000
(51) Int. Cl.: C12N 15/60, C12N 15/53, C12N 9/88, C12N 1/21, C12P 13/08, C12P 13/04, C12R 1/15

(54) **Coryneforme Bakterien mit einer Deletion der Phosphoenolpyruvat-Carboxykinase und ihre Verwendung**
Coryneform Bacteria with a deletion of Phosphoenolpyruvate-carboxykinase and their use
Mutant de délétion de phosphoenolpyruvate-carboxykinase de bactérie coryneforme et son usage

(30) Priorität: 20.10.1999 DE 19950409
(43) Veröffentlichungstag der Anmeldung: 25.04.2001
(73) Patentinhaber: Degussa AG, 40474 Düsseldorf (DE); Forschungszentrum Jülich GmbH, 52425 Jülich (DE)
(72) Erfinder: Eikmanns, Bernhard, Prof. Dr., 89081 Ulm (DE); Riedel, Christian, 89233 Neu-Ulm (DE); Sahm, Hermann, Prof., 52428 Jülich (DE); Möckel, Bettina, Dr., 40597 Düsseldorf (DE)

(56) Entgegenhaltungen:
- EP-A- 0 358 940
- WO-A-01/00844
- DATABASE EMBL [Online] AC S56812, Chlorobium limicola, PEPCK, XP002167027
- KRAMER R: "Genetic and physiological approaches for the production of amino acids" JOURNAL OF BIOTECHNOLOGY,NL,ELSEVIER SCIENCE PUBLISHERS, AMSTERDAM, Bd. 45, Nr. 1, 12. Februar 1996 (1996-02-12), Seiten 1-21, XP004036833 ISSN: 0168-1656
- BERNHARD J EIKMANNS ET AL: "A family of Corynebacterium glutamicum/Escherichia coli shuttle vectors for cloning, controlled gene expression, and promoter probing" GENE: AN INTERNATIONAL JOURNAL ON GENES AND GENOMES,ELSEVIER SCIENCE PUBLISHERS, BARKING,GB, Bd. 102, 1991, Seiten 93-98, XP002153371 ISSN: 0378-1119
- DATABASE BIOSIS [Online] BIOSCIENCES INFORMATION SERVICE, PHILADELPHIA, PA, US; PREV199396116299, JETTEN M., AND SINSKEY A.J.: "Characterization of PEP carboxykinase from C. glutamicum" XP002167028

### SEQUENZPROTOKOLL

<110> Degussa-Hüls AG
   Forschungszentrum Jülich GmbH
<120> Neue für das pck-Gen codierende Nukleotidsequenzen.
<130> 990110BT
<140>
   <141>
<160> 2
<170> PatentIn Ver. 2.1
<210> 1
   <211> 3935
   <212> DNA
   <213> Corynebacterium glutamicum
<220>
   <221> CDS
   <222> (2022)..(3851)
<400> 1
   ctggcagttc tcctaattga tcgcgggaat tatcagaaat agacattatt tgttattttt 60
   cctgttcaac tttaaaactt caatattcgt gagtttggat gaatccctag agcactacct 120
   tttagacctc tcgctgcaat ttaggccagt tgagatttaa gctttccgac gattcttctc 180
   attactgcaa tcgtaccggc gatggtggac acgatgacat gaaagagcat taaagcaatc 240
   aagtacaggc tgaagtagtt aaaccactcc actccggtgc tctgtgataa aaaatgcgca 300
   cccaaactca aagtgccaac tgggaaggta ctggcccacc atgtggggct gtatgtcgcc 360
   cctttgaaaa cagctctgta gaacacaaag tgagcgatgg ctcccagagg aatcgtaaaa 420
   attcccatga tgatgccgta aataatgccc attgtgattg ctgtcttgga tccaaaggac 480
   gcaccgatga gctgagctgc tgcagtggat tggcccacca tacccaaagg aatccatgat 540
   gttggtgttg ccatcagtgg gatgccctgc gccttggggc cgaaatagta gaaatacact 600
   cgggtaaaaa ctgctggtgc agacgccaaa gttaaaagga agagcccgaa agaaacccac 660
   agcatcgccg gaagttcaaa gtgctcatgg agttgtgctg ccgaggtgga agcaaccatc 720
   ggcgtgacaa gaggaagacc ccacgcaaaa gttggtgtgc ccgccttaga tcgcaaaatg 780
   gccgttatat ataaggaata ggcaacaagt cccacggctg tgccaataga ccagcacaca 840
   aacataaatc cccacagatc atcacccaaa actacggggc ttgcagttcc caatgcgatc 900
   aaacccatgg acagcattgc ccatgccggc atgacttcag ttttgaatga aggagagcgg 960
   tagattagcc aaccgccaat aatgacaatt gccaccacaa cagctaacgc gaagaagaaa 1020
   tctgcgacga ctggaaaacc atggattttc aacagtgatg acaacaatga gatgcccatg 1080
   agggaaccag cccacgaggg gccaggtgga ggtaagaccg cagcgtagct tttggtcgaa 1140
   gaaggagtgg gcatgcccat tactttaagc ctttggggca gtgaaaccgc taaatgggag 1200
   cgttgtgcgc tcgatcactg gtctagacct ttgggctcca aaagttgcaa tttcgcgaat 1260
   acttcaacac ttgtttgcaa tgtttgttaa taaatgggtt cgctagtgga ttctgtcgtt 1320
   agtactggcc gtcgtggtgg ggtcatgtat ttaggtaggg caaagttaag atcagagcac 1380
   tttttgatac gactaactgg atataacctt tggggtaacg tggggatgtg tgtgagtaat 1440
   tttcaaagta tttaaaaggg ggatctaggg taaaaatttg gcttcaagta catatcttta 1500
   gttcggtagt tgagggcggg tggtgacagt gcgggcatgc atgtgagtgt aaatgttgtt 1560
   ttaaaaaggt gtgtactgac agtgggccgg tttgtgctgg tcggccacta gcggagtgct 1620
   tggattgtga tggcagggta agggaaaggg attaccatta ccgctgttct tggcgttttg 1680
   ttgcctattg tccgaatgtt aagtgttaat ggtgggaaaa ctgggaaagt tgtcccctgg 1740
   aatgtgtgag aattgcccaa atctgaaccc aatggccatg gacggggaat gaactgtcgg 1800
   agaacggttg aggttaattc ttgaaaccac ccccaaaata ggctatttaa acgggtgctc 1860
   tcatattaaa gaaagtgtgt agatgcgtgt gggcaggggg taggtccact ggtaatgaca 1920
   aatgtgtccg ttgtctcacc taaagtttta actagttctg tatctgaaag ctacgctagg 1980
   gggcgagaac tctgtcgaat gacacaaaat ctggagaagt a atg act act gct gca 2036
                                                 Met Thr Thr Ala Ala
                                                 1               5
   atc agg ggc ctt cag ggc gag gcg ccg acc aag aat aag gaa ctg ctg   2084
   Ile Arg Gly Leu Gln Gly Glu Ala Pro Thr Lys Asn Lys Glu Leu Leu
                   10                  15                  20
   aac tgg atc gca gac gcc gtc gag ctc ttc cag cct gag gct gtt gtg   2132
   Asn Trp Ile Ala Asp Ala Val Glu Leu Phe Gln Pro Glu Ala Val Val
               25                  30                  35
   ttc gtt gat gga tcc cag gct gag tgg gat cgc atg gcg gag gat ctt   2180
   Phe Val Asp Gly Ser Gln Ala Glu Trp Asp Arg Met Ala Glu Asp Leu
           40                  45                  50
   gtt gaa gcc ggt acc ctc atc aag ctc aac gag gaa aag cgt ccg aac   2228
   Val Glu Ala Gly Thr Leu Ile Lys Leu Asn Glu Glu Lys Arg Pro Asn
       55                  60                  65
   agc tac cta gct cgt tcc aac cca tct gac gtt gcg cgc gtt gag tcc   2276
   Ser Tyr Leu Ala Arg Ser Asn Pro Ser Asp Val Ala Arg Val Glu Ser
   70                  75                  80                  85
   cgc acc ttc atc tgc tcc gag aag gaa gaa gat gct ggc cca acc aac    2324
   Arg Thr Phe Ile Cys Ser Glu Lys Glu Glu Asp Ala Gly Pro Thr Asn
                       90                  95                 100
   aac tgg gct cca cca cag gca atg aag gac gaa atg tcc aag cat tac    2372
   Asn Trp Ala Pro Pro Gln Ala Met Lys Asp Glu Met Ser Lys His Tyr
               105                 110                 115
   gct ggt tcc atg aag ggg cgc acc atg tac gtc gtg cct ttc tgc atg    2420
   Ala Gly Ser Met Lys Gly Arg Thr Met Tyr Val Val Pro Phe Cys Met
           120                 125                 130
   ggt cca atc agc gat ccg gac cct aag ctt ggt gtg cag ctc act gac    2468
   Gly Pro Ile Ser Asp Pro Asp Pro Lys Leu Gly Val Gln Leu Thr Asp
       135                 140                 145
   tcc gag tac gtt gtc atg tcc atg cgc atc atg acc cgc atg ggt att    2516
   Ser Glu Tyr Val Val Met Ser Met Arg Ile Met Thr Arg Met Gly Ile
   150                 155                 160                 165
   gaa gcg ctg gac aag atc ggc gcg aac ggc agc ttc gtc agg tgc ctc    2564
   Glu Ala Leu Asp Lys Ile Gly Ala Asn Gly Ser Phe Val Arg Cys Leu
                   170                 175                 180
   cac tcc gtt ggt gct cct ttg gag cca ggc cag gaa gac gtt gca tgg    2612
   His Ser Val Gly Ala Pro Leu Glu Pro Gly Gln Glu Asp Val Ala Trp
               185                 190                 195
   cct tgc aac gac acc aag tac atc acc cag ttc cca gag acc aag gaa    2660
   Pro Cys Asn Asp Thr Lys Tyr Ile Thr Gln Phe Pro Glu Thr Lys Glu
           200                 205                 210
   att tgg tcc tac ggt tcc ggc tac ggc gga aac gca atc ctg gca aag    2708
   Ile Trp Ser Tyr Gly Ser Gly Tyr Gly Gly Asn Ala Ile Leu Ala Lys
       215                 220                 225
   aag tgc tac gca ctg cgt atc gca tct gtc atg gct cgc gaa gaa gga    2756
   Lys Cys Tyr Ala Leu Arg Ile Ala Ser Val Met Ala Arg Glu Glu Gly
   230                 235                 240                 245
   tgg atg gct gag cac atg ctc atc ctg aag ctg atc aac cca gag ggc    2804
   Trp Met Ala Glu His Met Leu Ile Leu Lys Leu Ile Asn Pro Glu Gly
                   250                 255                 260
   aag gcg tac cac atc gca gca gca ttc cca tct gct tgt ggc aag acc    2852
   Lys Ala Tyr His Ile Ala Ala Ala Phe Pro Ser Ala Cys Gly Lys Thr
               265                 270                 275
   aac ctc gcc atg atc act cca acc atc cca ggc tgg acc gct cag gtt    2900
   Asn Leu Ala Met Ile Thr Pro Thr Ile Pro Gly Trp Thr Ala Gln Val
           280                 285                 290
   gtt ggc gac gac atc gct tgg ctg aag ctg cgc gag gac ggc ctc tac    2948
   Val Gly Asp Asp Ile Ala Trp Leu Lys Leu Arg Glu Asp Gly Leu Tyr
       295                 300                 305
   gca gtt aac cca gaa aat ggt ttc ttc ggt gtt gct cca ggc acc aac    2996
   Ala Val Asn Pro Glu Asn Gly Phe Phe Gly Val Ala Pro Gly Thr Asn
   310                 315                 320                 325
   tac gca tcc aac cca atc gcg atg aag acc atg gaa cca ggc aac acc    3044
   Tyr Ala Ser Asn Pro Ile Ala Met Lys Thr Met Glu Pro Gly Asn Thr
                   330                 335                 340
   ctg ttc acc aac gtg gca ctc acc gac gac ggc gac atc tgg tgg gaa    3092
   Leu Phe Thr Asn Val Ala Leu Thr Asp Asp Gly Asp Ile Trp Trp Glu
               345                 350                 355
   ggc atg gac ggc gac gcc cca gct cac ctc att gac tgg atg ggc aac    3140
   Gly Met Asp Gly Asp Ala Pro Ala His Leu Ile Asp Trp Met Gly Asn
           360                 365                 370
   gac tgg acc cca gag tcc gac gaa aac gct gct cac cct aac tcc cgt    3188
   Asp Trp Thr Pro Glu Ser Asp Glu Asn Ala Ala His Pro Asn Ser Arg
       375                 380                 385
   tac tgc gta gca atc gac cag tcc cca gca gca gca cct gag ttc aac    3236
   Tyr Cys Val Ala Ile Asp Gln Ser Pro Ala Ala Ala Pro Glu Phe Asn
   390                 395                 400                 405
   gac tgg gaa ggc gtc aag atc gac gca atc ctc ttc ggt gga cgt cgc    3284
   Asp Trp Glu Gly Val Lys Ile Asp Ala Ile Leu Phe Gly Gly Arg Arg
                   410                 415                 420
   gca gac acc gtc cca ctg gtt acc cag acc tac gac tgg gag cac ggc    3332
   Ala Asp Thr Val Pro Leu Val Thr Gln Thr Tyr Asp Trp Glu His Gly
               425                 430                 435
   acc atg gtt ggt gca ctg ctc gca tcc ggt cag acc gca gct tcc gca    3380
   Thr Met Val Gly Ala Leu Leu Ala Ser Gly Gln Thr Ala Ala Ser Ala
           440                 445                 450
   gaa gca aag gtc ggc aca ctc cgc cac gac cca atg gca atg ctc cca    3428
   Glu Ala Lys Val Gly Thr Leu Arg His Asp Pro Met Ala Met Leu Pro
       455                 460                 465
   ttc att ggc tac aac gct ggt gaa tac ctg cag aac tgg att gac atg    3476
   Phe Ile Gly Tyr Asn Ala Gly Glu Tyr Leu Gln Asn Trp Ile Asp Met
   470                 475                 480                 485
   ggt aac aag ggt ggc gac aag atg cca tcc atc ttc ctg gtc aac tgg    3524
   Gly Asn Lys Gly Gly Asp Lys Met Pro Ser Ile Phe Leu Val Asn Trp
                   490                 495                 500
   ttc cgc cgt ggc gaa gat gga cgc ttc ctg tgg cct ggc ttc ggc gac    3572
   Phe Arg Arg Gly Glu Asp Gly Arg Phe Leu Trp Pro Gly Phe Gly Asp
               505                 510                 515
   aac tct cgc gtt ctg aag tgg gtc atc gac cgc atc gaa ggc cac gtt    3620
   Asn Ser Arg Val Leu Lys Trp Val Ile Asp Arg Ile Glu Gly His Val
           520                 525                 530
   ggc gca gac gag acc gtt gtt gga cac acc gct aag gcc gaa gac ctc   3668
   Gly Ala Asp Glu Thr Val Val Gly His Thr Ala Lys Ala Glu Asp Leu
       535                 540                 545
   gac ctc gac ggc ctc gac acc cca att gag gat gtc aag gaa gca ctg   3716
   Asp Leu Asp Gly Leu Asp Thr Pro Ile Glu Asp Val Lys Glu Ala Leu
   550                 555                 560                 565
   acc gct cct gca gag cag tgg gca aac gac gtt gaa gac aac gcc gag   3764
   Thr Ala Pro Ala Glu Gln Trp Ala Asn Asp Val Glu Asp Asn Ala Glu
                   570                 575                 580
   tac ctc act ttc ctc gga cca cgt gtt cct gca gag gtt cac agc cag   3812
   Tyr Leu Thr Phe Leu Gly Pro Arg Val Pro Ala Glu Val His Ser Gln
               585                 590                 595
   ttc gat gct ctg aag gcc cgc att tca gca gct cac gct taaagttcac    3861
   Phe Asp Ala Leu Lys Ala Arg Ile Ser Ala Ala His Ala
           600                 605                 610
   gcttaagaac tgctaaataa caagaaaggc tcccaccgaa agtgggagcc tttcttgtcg 3921
   ttaagcgatg aatt                                                   3935
<210> 2
   <211> 610
   <212> PRT
   <213> Corynebacterium glutamicum
<400> 2
   Met Thr Thr Ala Ala Ile Arg Gly Leu Gln Gly Glu Ala Pro Thr Lys
   1               5                   10                  15
   Asn Lys Glu Leu Leu Asn Trp Ile Ala Asp Ala Val Glu Leu Phe Gln
               20                  25                  30
   Pro Glu Ala Val Val Phe Val Asp Gly Ser Gln Ala Glu Trp Asp Arg
           35                  40                  45
   Met Ala Glu Asp Leu Val Glu Ala Gly Thr Leu Ile Lys Leu Asn Glu
       50                  55                  60
   Glu Lys Arg Pro Asn Ser Tyr Leu Ala Arg Ser Asn Pro Ser Asp Val
   65                  70                  75                  80
   Ala Arg Val Glu Ser Arg Thr Phe Ile Cys Ser Glu Lys Glu Glu Asp
                   85                  90                  95
   Ala Gly Pro Thr Asn Asn Trp Ala Pro Pro Gln Ala Met Lys Asp Glu
               100                 105                 110
   Met Ser Lys His Tyr Ala Gly Ser Met Lys Gly Arg Thr Met Tyr Val
           115                 120                 125
   Val Pro Phe Cys Met Gly Pro Ile Ser Asp Pro Asp Pro Lys Leu Gly
       130                 135                 140
   Val Gln Leu Thr Asp Ser Glu Tyr Val Val Met Ser Met Arg Ile Met
   145 150 155 160
   Thr Arg Met Gly Ile Glu Ala Leu Asp Lys Ile Gly Ala Asn Gly Ser
   165 170 175
   Phe Val Arg Cys Leu His Ser Val Gly Ala Pro Leu Glu Pro Gly Gln
   180 185 190
   Glu Asp Val Ala Trp Pro Cys Asn Asp Thr Lys Tyr Ile Thr Gln Phe
   195 200 205
   Pro Glu Thr Lys Glu Ile Trp Ser Tyr Gly Ser Gly Tyr Gly Gly Asn
   210 215 220
   Ala Ile Leu Ala Lys Lys Cys Tyr Ala Leu Arg Ile Ala Ser Val Met
   225 230 235 240
   Ala Arg Glu Glu Gly Trp Met Ala Glu His Met Leu Ile Leu Lys Leu
   245 250 255
   Ile Asn Pro Glu Gly Lys Ala Tyr His Ile Ala Ala Ala Phe Pro Ser
   260 265 270
   Ala Cys Gly Lys Thr Asn Leu Ala Met Ile Thr Pro Thr Ile Pro Gly
   275 280 285
   Trp Thr Ala Gln Val Val Gly Asp Asp Ile Ala Trp Leu Lys Leu Arg
   290 295 300
   Glu Asp Gly Leu Tyr Ala Val Asn Pro Glu Asn Gly Phe Phe Gly Val
   305 310 315 320
   Ala Pro Gly Thr Asn Tyr Ala Ser Asn Pro Ile Ala Met Lys Thr Met
   325 330 335
   Glu Pro Gly Asn Thr Leu Phe Thr Asn Val Ala Leu Thr Asp Asp Gly
   340 345 350
   Asp Ile Trp Trp Glu Gly Met Asp Gly Asp Ala Pro Ala His Leu Ile
   355 360 365
   Asp Trp Met Gly Asn Asp Trp Thr Pro Glu Ser Asp Glu Asn Ala Ala
   370 375 380
   His Pro Asn Ser Arg Tyr Cys Val Ala Ile Asp Gln Ser Pro Ala Ala
   385 390 395 400
   Ala Pro Glu Phe Asn Asp Trp Glu Gly Val Lys Ile Asp Ala Ile Leu
   405 410 415
   Phe Gly Gly Arg Arg Ala Asp Thr Val Pro Leu Val Thr Gln Thr Tyr
   420 425 430
   Asp Trp Glu His Gly Thr Met Val Gly Ala Leu Leu Ala Ser Gly Gln
   435 440 445
   Thr Ala Ala Ser Ala Glu Ala Lys Val Gly Thr Leu Arg His Asp Pro
   450 455 460
   Met Ala Met Leu Pro Phe Ile Gly Tyr Asn Ala Gly Glu Tyr Leu Gln
   465                 470                 475                 480
   Asn Trp Ile Asp Met Gly Asn Lys Gly Gly Asp Lys Met Pro Ser Ile
                   485                 490                 495
   Phe Leu Val Asn Trp Phe Arg Arg Gly Glu Asp Gly Arg Phe Leu Trp
               500                 505                 510
   Pro Gly Phe Gly Asp Asn Ser Arg Val Leu Lys Trp Val Ile Asp Arg
           515                 520                 525
   Ile Glu Gly His Val Gly Ala Asp Glu Thr Val Val Gly His Thr Ala
       530                 535                 540
   Lys Ala Glu Asp Leu Asp Leu Asp Gly Leu Asp Thr Pro Ile Glu Asp
   545                 550                 555                 560
   Val Lys Glu Ala Leu Thr Ala Pro Ala Glu Gln Trp Ala Asn Asp Val
                   565                 570                 575
   Glu Asp Asn Ala Glu Tyr Leu Thr Phe Leu Gly Pro Arg Val Pro Ala
               580                 585                 590
   Glu Val His Ser Gln Phe Asp Ala Leu Lys Ala Arg Ile Ser Ala Ala
           595                 600                 605
   His Ala
       610

### SEQUENZPROTOKOLL

<110> Degussa-Hüls AG
   Forschungszentrum Jülich GmbH
<120> Neue für das pck-Gen codierende Nukleotidsequenzen.
<130> 990110BT
<140>
   <141>
<160> 2
<170> PatentIn Ver. 2.1
<210> 1
   <211> 3935
   <212> DNA
   <213> Corynebacterium glutamicum
<220>
   <221> CDS
   <222> (2022)..(3851)
<400> 1
   ctggcagttc tcctaattga tcgcgggaat tatcagaaat agacattatt tgttattttt 60
   cctgttcaac tttaaaactt caatattcgt gagtttggat gaatccctag agcactacct 120
   tttagacctc tcgctgcaat ttaggccagt tgagatttaa gctttccgac gattcttctc 180
   attactgcaa tcgtaccggc gatggtggac acgatgacat gaaagagcat taaagcaatc 240
   aagtacaggc tgaagtagtt aaaccactcc actccggtgc tctgtgataa aaaatgcgca 300
   cccaaactca aagtgccaac tgggaaggta ctggcccacc atgtggggct gtatgtcgcc 360
   cctttgaaaa cagctctgta gaacacaaag tgagcgatgg ctcccagagg aatcgtaaaa 420
   attcccatga tgatgccgta aataatgccc attgtgattg ctgtcttgga tccaaaggac 480
   gcaccgatga gctgagctgc tgcagtggat tggcccacca tacccaaagg aatccatgat 540
   gttggtgttg ccatcagtgg gatgccctgc gccttggggc cgaaatagta gaaatacact 600
   cgggtaaaaa ctgctggtgc agacgccaaa gttaaaagga agagcccgaa agaaacccac 660
   agcatcgccg gaagttcaaa gtgctcatgg agttgtgctg ccgaggtgga agcaaccatc 720
   ggcgtgacaa gaggaagacc ccacgcaaaa gttggtgtgc ccgccttaga tcgcaaaatg 780
   gccgttatat ataaggaata ggcaacaagt cccacggctg tgccaataga ccagcacaca 840
   aacataaatc cccacagatc atcacccaaa actacggggc ttgcagttcc caatgcgatc 900
   aaacccatgg acagcattgc ccatgccggc atgacttcag ttttgaatga aggagagcgg 960
   tagattagcc aaccgccaat aatgacaatt gccaccacaa cagctaacgc gaagaagaaa 1020
   tctgcgacga ctggaaaacc atggattttc aacagtgatg acaacaatga gatgcccatg 1080
   agggaaccag cccacgaggg gccaggtgga ggtaagaccg cagcgtagct tttggtcgaa 1140
   gaaggagtgg gcatgcccat tactttaagc ctttggggca gtgaaaccgc taaatgggag 1200
   cgttgtgcgc tcgatcactg gtctagacct ttgggctcca aaagttgcaa tttcgcgaat 1260
   acttcaacac ttgtttgcaa tgtttgttaa taaatgggtt cgctagtgga ttctgtcgtt 1320
   agtactggcc gtcgtggtgg ggtcatgtat ttaggtaggg caaagttaag atcagagcac 1380
   tttttgatac gactaactgg atataacctt tggggtaacg tggggatgtg tgtgagtaat 1440
   tttcaaagta tttaaaaggg ggatctaggg taaaaatttg gcttcaagta catatcttta 1500
   gttcggtagt tgagggcggg tggtgacagt gcgggcatgc atgtgagtgt aaatgttgtt 1560
   ttaaaaaggt gtgtactgac agtgggccgg tttgtgctgg tcggccacta gcggagtgct 1620
   tggattgtga tggcagggta agggaaaggg attaccatta ccgctgttct tggcgttttg 1680
   ttgcctattg tccgaatgtt aagtgttaat ggtgggaaaa ctgggaaagt tgtcccctgg 1740
   aatgtgtgag aattgcccaa atctgaaccc aatggccatg gacggggaat gaactgtcgg 1800
   agaacggttg aggttaattc ttgaaaccac ccccaaaata ggctatttaa acgggtgctc 1860
   tcatattaaa gaaagtgtgt agatgcgtgt gggcaggggg taggtccact ggtaatgaca 1920
   aatgtgtccg ttgtctcacc taaagtttta actagttctg tatctgaaag ctacgctagg 1980
   gggcgagaac tctgtcgaat gacacaaaat ctggagaagt a atg act act gct gca 2036
                                                 Met Thr Thr Ala Ala
                                                  1               5
   atc agg ggc ctt cag ggc gag gcg ccg acc aag aat aag gaa ctg ctg   2084
   Ile Arg Gly Leu Gln Gly Glu Ala Pro Thr Lys Asn Lys Glu Leu Leu
                    10                  15                  20
   aac tgg atc gca gac gcc gtc gag ctc ttc cag cct gag gct gtt gtg   2132
   Asn Trp Ile Ala Asp Ala Val Glu Leu Phe Gln Pro Glu Ala Val Val
                25                  30                  35
   ttc gtt gat gga tcc cag gct gag tgg gat cgc atg gcg gag gat ctt   2180
   Phe Val Asp Gly Ser Gln Ala Glu Trp Asp Arg Met Ala Glu Asp Leu
            40                  45                  50
   gtt gaa gcc ggt acc ctc atc aag ctc aac gag gaa aag cgt ccg aac   2228
   Val Glu Ala Gly Thr Leu Ile Lys Leu Asn Glu Glu Lys Arg Pro Asn
        55                  60                  65
   agc tac cta gct cgt tcc aac cca tct gac gtt gcg cgc gtt gag tcc   2276
   Ser Tyr Leu Ala Arg Ser Asn Pro Ser Asp Val Ala Arg Val Glu Ser
    70                  75                  80                  85
   cgc acc ttc atc tgc tcc gag aag gaa gaa gat gct ggc cca acc aac     2324
   Arg Thr Phe Ile Cys Ser Glu Lys Glu Glu Asp Ala Gly Pro Thr Asn
                    90                  95                  100
   aac tgg gct cca cca cag gca atg aag gac gaa atg tcc aag cat tac     2372
   Asn Trp Ala Pro Pro Gln Ala Met Lys Asp Glu Met Ser Lys His Tyr
               105                 110                 115
   gct ggt tcc atg aag ggg cgc acc atg tac gtc gtg cct ttc tgc atg     2420
   Ala Gly Ser Met Lys Gly Arg Thr Met Tyr Val Val Pro Phe Cys Met
           120                 125                 130
   ggt cca atc agc gat ccg gac cct aag ctt ggt gtg cag ctc act gac     2468
   Gly Pro Ile Ser Asp Pro Asp Pro Lys Leu Gly Val Gln Leu Thr Asp
       135                 140                 145
   tcc gag tac gtt gtc atg tcc atg cgc atc atg acc cgc atg ggt att     2516
   Ser Glu Tyr Val Val Met Ser Met Arg Ile Met Thr Arg Met Gly Ile
   150                 155                 160                 165
   gaa gcg ctg gac aag atc ggc gcg aac ggc agc ttc gtc agg tgc ctc     2564
   Glu Ala Leu Asp Lys Ile Gly Ala Asn Gly Ser Phe Val Arg Cys Leu
                   170                 175                 180
   cac tcc gtt ggt gct cct ttg gag cca ggc cag gaa gac gtt gca tgg     2612
   His Ser Val Gly Ala Pro Leu Glu Pro Gly Gln Glu Asp Val Ala Trp
               185                 190                 195
   cct tgc aac gac acc aag tac atc acc cag ttc cca gag acc aag gaa     2660
   Pro Cys Asn Asp Thr Lys Tyr Ile Thr Gln Phe Pro Glu Thr Lys Glu
           200                 205                 210
   att tgg tcc tac ggt tcc ggc tac ggc gga aac gca atc ctg gca aag     2708
   Ile Trp Ser Tyr Gly Ser Gly Tyr Gly Gly Asn Ala Ile Leu Ala Lys
       215                 220                 225
   aag tgc tac gca ctg cgt atc gca tct gtc atg gct cgc gaa gaa gga     2756
   Lys Cys Tyr Ala Leu Arg Ile Ala Ser Val Met Ala Arg Glu Glu Gly
   230                 235                 240                 245
   tgg atg gct gag cac atg ctc atc ctg aag ctg atc aac cca gag ggc     2804
   Trp Met Ala Glu His Met Leu Ile Leu Lys Leu Ile Asn Pro Glu Gly
                   250                 255                 260
   aag gcg tac cac atc gca gca gca ttc cca tct gct tgt ggc aag acc     2852
   Lys Ala Tyr His Ile Ala Ala Ala Phe Pro Ser Ala Cys Gly Lys Thr
               265                 270                 275
   aac ctc gcc atg atc act cca acc atc cca ggc tgg acc gct cag gtt     2900
   Asn Leu Ala Met Ile Thr Pro Thr Ile Pro Gly Trp Thr Ala Gln Val
           280                 285                 290
   gtt ggc gac gac atc gct tgg ctg aag ctg cgc gag gac ggc ctc tac     2948
   Val Gly Asp Asp Ile Ala Trp Leu Lys Leu Arg Glu Asp Gly Leu Tyr
       295                 300                 305
   gca gtt aac cca gaa aat ggt ttc ttc ggt gtt gct cca ggc acc aac     2996
   Ala Val Asn Pro Glu Asn Gly Phe Phe Gly Val Ala Pro Gly Thr Asn
   310                 315                 320                 325
   tac gca tcc aac cca atc gcg atg aag acc atg gaa cca ggc aac acc     3044
   Tyr Ala Ser Asn Pro Ile Ala Met Lys Thr Met Glu Pro Gly Asn Thr
                   330                 335                 340
   ctg ttc acc aac gtg gca ctc acc gac gac ggc gac atc tgg tgg gaa     3092
   Leu Phe Thr Asn Val Ala Leu Thr Asp Asp Gly Asp Ile Trp Trp Glu
               345                 350                 355
   ggc atg gac ggc gac gcc cca gct cac ctc att gac tgg atg ggc aac     3140
   Gly Met Asp Gly Asp Ala Pro Ala His Leu Ile Asp Trp Met Gly Asn
           360                 365                 370
   gac tgg acc cca gag tcc gac gaa aac gct gct cac cct aac tcc cgt     3188
   Asp Trp Thr Pro Glu Ser Asp Glu Asn Ala Ala His Pro Asn Ser Arg
       375                 380                 385
   tac tgc gta gca atc gac cag tcc cca gca gca gca cct gag ttc aac     3236
   Tyr Cys Val Ala Ile Asp Gln Ser Pro Ala Ala Ala Pro Glu Phe Asn
   390                 395                 400                 405
   gac tgg gaa ggc gtc aag atc gac gca atc ctc ttc ggt gga cgt cgc     3284
   Asp Trp Glu Gly Val Lys Ile Asp Ala Ile Leu Phe Gly Gly Arg Arg
                   410                 415                 420
   gca gac acc gtc cca ctg gtt acc cag acc tac gac tgg gag cac ggc     3332
   Ala Asp Thr Val Pro Leu Val Thr Gln Thr Tyr Asp Trp Glu His Gly
               425                 430                 435
   acc atg gtt ggt gca ctg ctc gca tcc ggt cag acc gca gct tcc gca     3380
   Thr Met Val Gly Ala Leu Leu Ala Ser Gly Gln Thr Ala Ala Ser Ala
           440                 445                 450
   gaa gca aag gtc ggc aca ctc cgc cac gac cca atg gca atg ctc cca     3428
   Glu Ala Lys Val Gly Thr Leu Arg His Asp Pro Met Ala Met Leu Pro
       455                 460                 465
   ttc att ggc tac aac gct ggt gaa tac ctg cag aac tgg att gac atg     3476
   Phe Ile Gly Tyr Asn Ala Gly Glu Tyr Leu Gln Asn Trp Ile Asp Met
   470                 475                 480                 485
   ggt aac aag ggt ggc gac aag atg cca tcc atc ttc ctg gtc aac tgg     3524
   Gly Asn Lys Gly Gly Asp Lys Met Pro Ser Ile Phe Leu Val Asn Trp
                   490                 495                 500
   ttc cgc cgt ggc gaa gat gga cgc ttc ctg tgg cct ggc ttc ggc gac     3572
   Phe Arg Arg Gly Glu Asp Gly Arg Phe Leu Trp Pro Gly Phe Gly Asp
               505                 510                 515
   aac tct cgc gtt ctg aag tgg gtc atc gac cgc atc gaa ggc cac gtt     3620
   Asn Ser Arg Val Leu Lys Trp Val Ile Asp Arg Ile Glu Gly His Val
           520                 525                 530
   ggc gca gac gag acc gtt gtt gga cac acc gct aag gcc gaa gac ctc   3668
   Gly Ala Asp Glu Thr Val Val Gly His Thr Ala Lys Ala Glu Asp Leu
       535                 540                 545
   gac ctc gac ggc ctc gac acc cca att gag gat gtc aag gaa gca ctg   3716
   Asp Leu Asp Gly Leu Asp Thr Pro Ile Glu Asp Val Lys Glu Ala Leu
   550                 555                 560                 565
   acc gct cct gca gag cag tgg gca aac gac gtt gaa gac aac gcc gag   3764
   Thr Ala Pro Ala Glu Gln Trp Ala Asn Asp Val Glu Asp Asn Ala Glu
                   570                 575                 580
   tac ctc act ttc ctc gga cca cgt gtt cct gca gag gtt cac agc cag   3812
   Tyr Leu Thr Phe Leu Gly Pro Arg Val Pro Ala Glu Val His Ser Gln
               585                 590                 595
   ttc gat gct ctg aag gcc cgc att tca gca gct cac gct taaagttcac    3861
   Phe Asp Ala Leu Lys Ala Arg Ile Ser Ala Ala His Ala
           600                 605                 610
   gcttaagaac tgctaaataa caagaaaggc tcccaccgaa agtgggagcc tttcttgtcg 3921
   ttaagcgatg aatt                                                   3935
<210> 2
   <211> 610
   <212> PRT
   <213> Corynebacterium glutamicum
<400> 2
   Met Thr Thr Ala Ala Ile Arg Gly Leu Gln Gly Glu Ala Pro Thr Lys
    1               5                  10                  15
   Asn Lys Glu Leu Leu Asn Trp Ile Ala Asp Ala Val Glu Leu Phe Gln
               20                  25                  30
   Pro Glu Ala Val Val Phe Val Asp Gly Ser Gln Ala Glu Trp Asp Arg
           35                  40                  45
   Met Ala Glu Asp Leu Val Glu Ala Gly Thr Leu Ile Lys Leu Asn Glu
       50                  55                  60
   Glu Lys Arg Pro Asn Ser Tyr Leu Ala Arg Ser Asn Pro Ser Asp Val
   65                  70                  75                  80
   Ala Arg Val Glu Ser Arg Thr Phe Ile Cys Ser Glu Lys Glu Glu Asp
                   85                  90                  95
   Ala Gly Pro Thr Asn Asn Trp Ala Pro Pro Gln Ala Met Lys Asp Glu
               100                 105                 110
   Met Ser Lys His Tyr Ala Gly Ser Met Lys Gly Arg Thr Met Tyr Val
           115                 120                 125
   Val Pro Phe Cys Met Gly Pro Ile Ser Asp Pro Asp Pro Lys Leu Gly
       130                 135                 140
   Val Gln Leu Thr Asp Ser Glu Tyr Val Val Met Ser Met Arg Ile Met
   145                 150                 155                 160
   Thr Arg Met Gly Ile Glu Ala Leu Asp Lys Ile Gly Ala Asn Gly Ser
                   165                 170                 175
   Phe Val Arg Cys Leu His Ser Val Gly Ala Pro Leu Glu Pro Gly Gln
               180                 185                 190
   Glu Asp Val Ala Trp Pro Cys Asn Asp Thr Lys Tyr Ile Thr Gln Phe
           195                 200                 205
   Pro Glu Thr Lys Glu Ile Trp Ser Tyr Gly Ser Gly Tyr Gly Gly Asn
       210                 215                 220
   Ala Ile Leu Ala Lys Lys Cys Tyr Ala Leu Arg Ile Ala Ser Val Met
   225                 230                 235                 240
   Ala Arg Glu Glu Gly Trp Met Ala Glu His Met Leu Ile Leu Lys Leu
                   245                 250                 255
   Ile Asn Pro Glu Gly Lys Ala Tyr His Ile Ala Ala Ala Phe Pro Ser
               260                 265                 270
   Ala Cys Gly Lys Thr Asn Leu Ala Met Ile Thr Pro Thr Ile Pro Gly
           275                 280                 285
   Trp Thr Ala Gln Val Val Gly Asp Asp Ile Ala Trp Leu Lys Leu Arg
       290                 295                 300
   Glu Asp Gly Leu Tyr Ala Val Asn Pro Glu Asn Gly Phe Phe Gly Val
   305                 310                 315                 320
   Ala Pro Gly Thr Asn Tyr Ala Ser Asn Pro Ile Ala Met Lys Thr Met
                   325                 330                 335
   Glu Pro Gly Asn Thr Leu Phe Thr Asn Val Ala Leu Thr Asp Asp Gly
               340                 345                 350
   Asp Ile Trp Trp Glu Gly Met Asp Gly Asp Ala Pro Ala His Leu Ile
           355                 360                 365
   Asp Trp Met Gly Asn Asp Trp Thr Pro Glu Ser Asp Glu Asn Ala Ala
       370                 375                 380
   His Pro Asn Ser Arg Tyr Cys Val Ala Ile Asp Gln Ser Pro Ala Ala
   385                 390                 395                 400
   Ala Pro Glu Phe Asn Asp Trp Glu Gly Val Lys Ile Asp Ala Ile Leu
                   405                 410                 415
   Phe Gly Gly Arg Arg Ala Asp Thr Val Pro Leu Val Thr Gln Thr Tyr
               420                 425                 430
   Asp Trp Glu His Gly Thr Met Val Gly Ala Leu Leu Ala Ser Gly Gln
           435                 440                 445
   Thr Ala Ala Ser Ala Glu Ala Lys Val Gly Thr Leu Arg His Asp Pro
       450                 455                 460
   Met Ala Met Leu Pro Phe Ile Gly Tyr Asn Ala Gly Glu Tyr Leu Gln
   465                 470                 475                 480
   Asn Trp Ile Asp Met Gly Asn Lys Gly Gly Asp Lys Met Pro Ser Ile
                   485                 490                 495
   Phe Leu Val Asn Trp Phe Arg Arg Gly Glu Asp Gly Arg Phe Leu Trp
               500                 505                 510
   Pro Gly Phe Gly Asp Asn Ser Arg Val Leu Lys Trp Val Ile Asp Arg
           515                 520                 525
   Ile Glu Gly His Val Gly Ala Asp Glu Thr Val Val Gly His Thr Ala
       530                 535                 540
   Lys Ala Glu Asp Leu Asp Leu Asp Gly Leu Asp Thr Pro Ile Glu Asp
   545                 550                 555                 560
   Val Lys Glu Ala Leu Thr Ala Pro Ala Glu Gln Trp Ala Asn Asp Val
                   565                 570                 575
   Glu Asp Asn Ala Glu Tyr Leu Thr Phe Leu Gly Pro Arg Val Pro Ala
               580                 585                 590
   Glu Val His Ser Gln Phe Asp Ala Leu Lys Ala Arg Ile Ser Ala Ala
           595                 600                 605
   His Ala
       610

## Beschreibung

Gegenstand der Erfindung sind coryneforme Bakterien, in denen die Expression von für das pck-Genprodukt kodierende Nukleotidsequenzen ausgeschaltet ist, und ein Verfahren zur fermentativen Herstellung von L-Aminosäuren, insbesondere L-Lysin und L-Threonin.

### Stand der Technik

Aminosäuren, insbesondere Lysin und Threonin finden in der Tierernährung, in der Lebensmittelindustrie, in der pharmazeutischen Industrie und in der Humanmedizin Anwendung.

Es ist bekannt, daß diese Stoffe durch Fermentation von Stämmen coryneformer Bakterien insbesondere Corynebacterium glutamicum hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Stoffwechselprodukte sind und die gewünschte Aminosäure produzieren.

Seit einigen Jahren werden ebenfalls Methoden der rekombinanten DNA-Technik zur Stammverbesserung von L-Aminosäure produzierenden Stämmen von Corynebacterium eingesetzt.

### Aufgabe der Erfindung

Die Erfinder haben sich zur Aufgabe gestellt, neue Maßnahmen zur verbesserten fermentativen Herstellung von Aminosäuren der Öffentlichkeit zur Verfügung zu stellen.

### Beschreibung der Erfindung

Aminosäuren, insbesondere L-Lysin und L-Threonin, finden in der Tierernährung, in der Lebensmittelindustrie, in der pharmazeutischen Industrie und in der Humanmedizin Anwendung. Es besteht daher ein allgemeines Interesse daran, neue verbesserte Verfahren zur Herstellung dieser Produkte bereitzustellen.

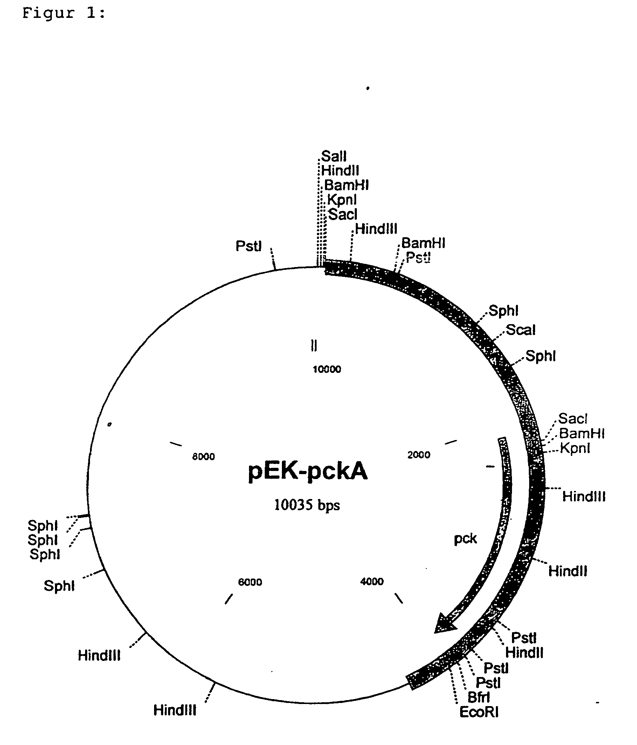

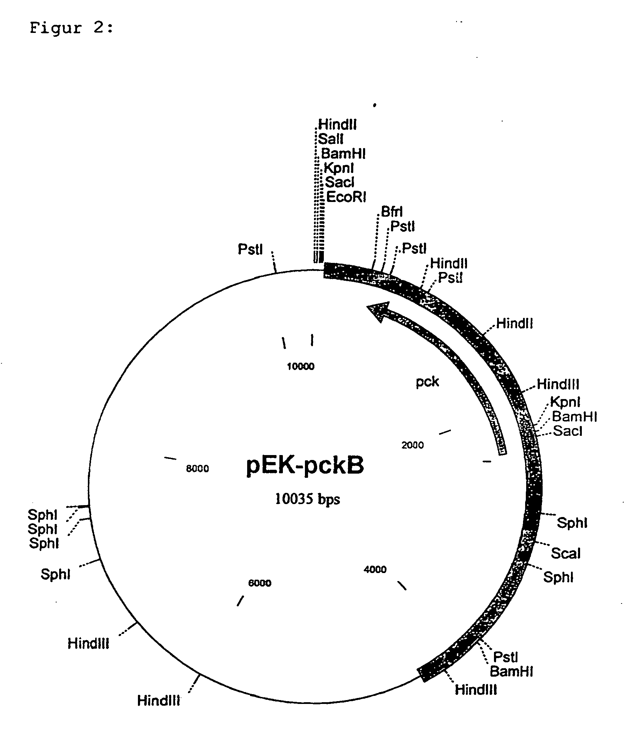

Die Beschreibung betrifft ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das mindestens zu 70 % identisch ist mit einem Polynukleotid, das für das genannte Polypeptid codiert und auf dem Plasmid pEK-pckA (Abb. 1) bzw. pEK-pckB (Abb. 2) enthalten ist,
c) Polynukleotid, das für ein Polypeptid codiert, das eine Aminosäuresequenz enthält, die zu mindestens 70 % identisch ist mit der Aminosäuresequenz von SEQ ID No. 2,
d) Polynukleotid, das komplementär ist zu den Polynukleotiden von a), b) oder c), und
e) Polynukleotid, enthaltend mindestens 15 aufeinanderfolgende Basen der Polynukleotidsequenz von a), b), c) oder d).

Gegenstand der Beschreibung, ist ebenso eine in coryneformen Mikroorganismen replizierbare, bevorzugt rekombinante DNA mit der Herkunft Corynebacterium, die zumindest die Nukleotidsequenz enthält, die für das pck-Gen, dargestellt in der SEQ ID No. 1, codiert.

Ebenfalls wird eine replizierbare DNA beschrieben enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz

(i) oder (ii) komplementären Sequenz hybridi siert, und/oder gegebenenfalls
(iv) funktionsneutralen Sinnmutationen in (i).

Ebenso werden beschrieben :
ein Polynukleotid enthaltend die Nukleotidsequenz wie in SEQ ID No. 1 dargestellt,
ein Polynukleotid, das für ein Polypeptid codiert, das die Aminosäuresequenz, wie in SEQ ID No. 2 dargestellt, enthält,
ein Vektor, enthaltend insbesondere pEK-pckA oder pEK-pckB, dargestellt in den Figuren 1 und 2
und als Wirtszelle dienende coryneforme Bakterien, in die die Δpck-Deletion eingebaut wurde.

Gegenstand der Beschreibung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthalten mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Beschreibung sind geeignet als Hybridisierungs-Sonden für RNA, cDNA und DNA, um cDNA in voller Länge zu isolieren, die für Phosphoenolpyruvat-Carboxykinase codieren und solche cDNA oder Gene zu isolieren, die eine hohe Ähnlichkeit der Sequenz mit der des Phosphoenolpyruvat-Carboxykinase Gens aufweisen.

Polynukleotidsequenzen gemäß der Beschreibung sind weiterhin als Primer zur Herstellung von DNA von Genen geeignet, die für Phosphoenolpyruvat-Carboxykinase codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Basen. Geeignet sind ebenfalls Oligonukleotide mit einer Länge von mindestens 40 oder 50 Basenpaaren.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA und DNA oder modifizierte RNA und DNA handeln kann.

Unter "Polypeptiden" versteht man Peptide oder Proteine, die zwei oder mehr über Peptidbindungen verbundene Aminosäuren erhalten.

Die Polypeptide gemäß Beschreibung, schließen das Polypeptid gemäß SEQ ID No. 2, insbesondere solche mit der biologischen Aktivität der PEP-Carboxykinase und auch solche ein, die zu wenigstens 70 % identisch sind mit dem Polypeptid gemäß SEQ ID No. 2, bevorzugt zu wenigstens 80 % und besonders solche, die zu wenigstens 90 % bis 95 % Identität zu dem Polypeptid gemäß SEQ ID No. 2 und die genannte Aktivität aufweisen.

Gegenstand der Erfindung ist ein isoliertes coryneformes Bakterium, bei dem die Expression eine Polynukleotids ausgeschaltet ist, das für ein Polypeptid kodiert, das eine Aminosäuresequenz hat, die zu mindestens 90 % identisch ist mit der Aminosäuresequenz von SEQ ID NO:2 , wobei das Polypeptid die Funktion einer Phosphoenolpyruvat-Carboxykinase hat.

Das genannte Bakterium gehört bevorzugt zur Gattung Corynebacterium, insbesondere zur Spezies Corynebacterium glutamicum.

Beansprucht wird auch ein Bakterium, bei dem das Polynukleotid ausgewählt ist aus der Gruppe:
a) der in SEQ ID NO:1 wiedergegebenen Nukleotidsequenz oder
b) Nukleotidsequenzen, die der Sequenz SEQ ID NO:1 innerhalb der Degeneration des genetischen Codes entsprechen, oder
c) Nukleotidsequenzen mit neutralen Sinnmutationen in SEQ ID NO:1.

Das genannte Polypeptid hat bevorzugt die in SEQ ID NO:2 wiedergegebene Aminosäuresequenz.

Die Ausschaltung wird bevorzugt durch Insertion oder Deletion von mindestens einem Basenpaar in dem genannten Polynukleotid erzielt.

Gegenstand der Erfindung sind ebenfalls der Vektor pk19mobsacBΔpck, hinterlegt in dem Stamm E. coli DH5α unter der Nummer DSM 13047 und als Wirtszelle dienende coryneforme Bakterien, die den genannten Vektor enthalten oder in die die Δpck-Deletion eingebaut wurde.

Die Erfindung betrifft weiterhin ein Verfahren zur fermentativen Herstellung von L-Aminosäuren, insbesondere L-Lysin und L-Threonin unter Verwendung der oben beschribenen coryneformen Bakterien, die insbesondere bereits die L-Aminosäuren produzieren und in denen die für das pck-Gen produkt codierend(en) Nukleotidsequenz(en) ausgeschaltet werden.

Beansprucht wird ebenso ein Verfahren zur Herstellung von L-Aminosäuren, umfassend das Fermentieren coryneformer Bakterien, in denen die intrazelluläre Aktivität des Polypeptids mit der in SEQ ID NO:2 wiedergegebenen Aminosäuresequenz ausgeschaltet ist, insbesondere die Expression der Polynukleotide mit den Sequenzen ausgeschaltet ist, ausgewählt aus der Gruppe:
a) der in SEQ ID NO:1 dargelegten Nukleotisequenz oder
b) Nukleotidsequenzen, die der SEQ ID NO: 1 innerhalb der Degeneration des genetischen Codes entsprechen, oder
c) Nukleotidsequenzen mit neutralen Sinnmutationen in SEQ ID NO: 1,
die für ein Polypeptid mit der Funktion einer Phosphoenolpyruvat-Carboxykinase kodieren.

Dieses Verfahren umfasst bevorzugt
a) die Fermentation der genannten coryneformen Bakterien, die die gewünschte L-Aminosäure produzieren,
b) die Konzentration der genannten L-Aminosäure in dem Medium oder in den Zellen der Bakterien und
c) das Isolieren der L-Aminosäure.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Enzym (Protein) inaktiviert und gegebenenfalls diese Maßnahmen kombiniert.

Die Mikroorganismen, die Gegenstand der vorliegenden Erfindung sind, können L-Aminosäuren insbesondere Lysin und Threonin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln.

Bei der Gattung Corynebacterium ist insbesondere die Art Corynebacterium glutamicum zu nennen, die in der Fachwelt für ihre Fähigkeit bekannt ist, L-Aminosäuren zu produzieren. '

Geeignete Stämme der Gattung Corynebacterium, insbesondere der Art Corynebacterium glutamicum, sind beispielsweise die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Aminosäure produzierende Mutanten bzw. Stämme,
wie beispielsweise die Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5714 oder
wie beispielsweise die L-Threonin produzierenden Stämme
Corynebacterium glutamicum ATCC21649
Brevibacterium flavum BB69
Brevibacterium flavum DSM5399
Brevibacterium lactofermentum FERM-BP 269
Brevibacterium lactofermentum TBB-10
Corynebacterium glutamicum MH20-22B-DR17.

Es gelang, das für das Enzym Phosphoenolpyruvat-Carboxykinase (PEP-Carboxykinase) (EC 4.1.1.49) kodierende pck-Gen von C. glutamicum zu isolieren.

Zur Isolierung des pck-Gens oder. auch anderer Gene von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Börmann et al. (Molecular Microbiology 6(3), 317-326)) wiederum beschreiben eine Genbank von C. glutamicum ATCC13032 unter Verwendung des Cosmids pHC79 (Hohn und Collins, Gene 11, 291-298 (1980)).

Zur Herstellung einer Genbank von C. glutamicum in E. coli können auch Plasmide bzw. Plasmidvektoren wie beispielsweise pBR322 (Bolivar, Life Sciences, 25, 807-818 (1979)), pUC9 (Vieira et al., 1982, Gene, 19:259-268), pACYC177 (Chang und Cohen, Journal of Bacteriology 134, 1141-1156 (1978)) oder pSC101 (Cohen und Chang, Journal of Bacteriology 132, 734-737 (1977)) verwendet werden. Als Wirte eignen sich besonders solche E. coli-Stämme, die restriktions- und rekombinationsdefekt sind

Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123:343-347) eingebaut. Der Indikatorstamm zeichnet sich dadurch aus, daß er eine Mutation in dem interessierenden Gen besitzt, die einen detektierbaren Phänotyp hervorruft. Im Rahmen der vorliegenden Erfindung ist die von Goldie und Sanwal (Journal of Bacteriology 141: 1115-1121 (1980)) beschriebene E. coli Mutante HG4 von Bedeutung. Dieser Stamm trägt eine Mutation im pck-Gen, wodurch das Wachstum auf Succinat als alleiniger Kohlenstoffquelle stark beeinträchtigt wird. Durch Transformation mit einem das pck-Gen enthaltenden Vektor kann das Wachstum auf Succinat wiederhergestellt werden.

Die mit Hilfe von Cosmiden oder anderen Vektoren klonierten langen DNA-Fragmente können anschließend in Form kürzerer DNA-Fragmente in bekannte Plasmidvektoren subkloniert werden. Dadurch wird die Zuordnung des erfindungsgemäßen Gens zu einem spezifischen DNA-Abschnitt ermöglicht. Hierzu verwendet man aus dem Stand der Technik bekannte Plasmidvektoren wie z. B. pBR322 (Bolivar, Life Sciences, 25, 807-818 (1979)) oder die von Bartolomé et al. (Gene 102, 75-78 (1991)) beschriebenen pSU-Vektoren. Vorzugsweise verwendet man jedoch Pendelvektoren, die sowohl in Escherichia coli als auch in Corynebacterium glutamicum replizieren, wie z. B. pZ1 (Menkel et al., Applied and Environmental Microbiology (1989) 64: 549-554) oder pEK0 (Eikmanns et al., Gene 102 (1991)), um Untersuchungen in beiden Spezies durchführen zu können. Beispiele hierfür sind die Plasmide pEK-pckA (Figur 1) und pEK-pckB (Figur 2), die ausgehend von dem Plasmidvektor pEK0 hergestellt wurden und das pck-Gen tragen.

Die auf diese Weise charakterisierten DNA-Abschnitte werden anschließend wiederum in gängige für die DNA-Sequenzierung geeignete Vektoren subkloniert. Alternativ können die langen in Cosmiden klonierten DNA-Abschnitte direkt in Sequenziervektoren subkloniert werden. Beispiele für derartige für die DNA-Sequenzierung geeignete Vektoren sind die Plasmide pGEM-5zf(-) oder pGEM-5zf(+) der Firma Promega Corporation (Promega Protocols ahd Application Guide, Second Edition, 1991, part number Y981, Promega Corporation, Madison, WI, USA).

Methoden zur DNA-Sequenzierung sind unter anderem bei Sanger et al. (Proceedings of the National of Sciences of the United States of America USA, 74:5463-5467, 1977) beschrieben.

Die erhaltenen DNA-Sequenzen können dann mit bekannten Algorithmen bzw. Sequenzanalyse-Programmen wie z. B. dem von Staden (Nucleic Acids Research 14, 217-232(1986)),dem GCG-Programm von Butler (Methods of Biochemical Analysis 39, 74-97 (1998)) dem FASTA-Algorithmus von Pearson und Lipman (Proceedings of the National Academy of Sciences USA 85,2444-2448 (1988)) oder dem BLAST-Algorithmus von Altschul et al. (Nature Genetics 6, 119-129 (1994)) untersucht und mit den in öffentlich zugänglichen Datenbanken vorhandenen Sequenzeinträgen verglichen werden. Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Bethesda, MD, USA).

Auf diese Weise wurde die für das pck-Gen produkt kodierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des pck-Genproduktes dargestellt.

Kodierende DNA-Sequenzen, die sich aus SEQ ID No. 1 durch die Degeneriertheit des genetischen Codes ergeben, sind ebenfalls Bestandteil der Beschreibung. In gleicher Weise sind DNA-Sequenzen, die mit SEQ ID No. 1 oder Teilen von SEQ ID No. 1 hybridisieren Bestandteil der Beschreibung. Schließlich sind DNA-Sequenzen Bestandteil der Beschreibung die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Basenpaaren.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Ausschaltung des pck-Gens in verbesserter Weise L-Aminosäuren insbesondere Lysin und Threonin produzieren.

Zur Erzielung einer Abschwächung kann entweder die Expression des pck-Gens oder die katalytischen Eigenschaften des Enzymproteins herabgesetzt bzw. ausgeschaltet werden. Gegebenenfalls können beide Maßnahmen kombiniert werden.

Die Erniedrigung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Patek et al. (Microbiology 142: 1297 (1996) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen (missense mutations) oder Nichtsinnmutationen (nonsense mutations) gesprochen. Insertionen oder Deletionen von mindestens einem Basenpaar in einem Gen führen zu Rasterverschiebungsmutationen (frame shift mutations), die dazu führen, daß falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

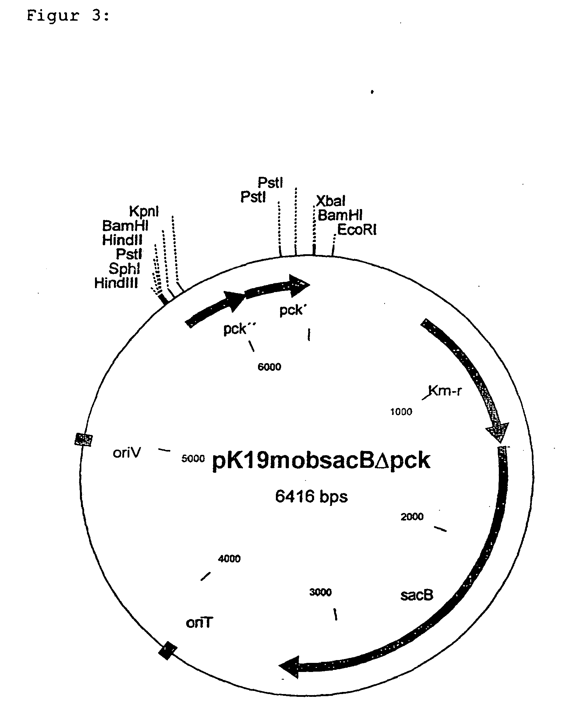

Ein Beispiel für ein mutiertes pck-Gen ist das in Plasmid pK19mobsacBΔpck (Figur 3) enthaltene Δpck-Allel. Das Δpck-Allel enthält lediglich die 5'- und die 3'-Flanke des pck-Gens; ein 1071 bp langer Abschnitt der Kodierregion fehlt (Deletion). Dieses Δpck-Allel kann durch Integrationsmutagenese in coryneforme Bakterien eingebaut werden. Hierzu bedient man sich des oben angegebenen Plasmides pK19mobsacBΔpck, das in C. glutamicum nicht replizierbar ist. Nach Übertragung durch Konjugation oder Transformation und homologer Rekombination mittels eines ersten, Integration bewirkenden "cross over"-Ereignisses und eines zweiten, eine Excision bewirkenden "cross over"-Ereignisses im pck-Gen erreicht man den Einbau des Δpck-Allels und erzielt einen Totalverlust der Enzymfunktion in dem jeweiligen Stamm.

Anleitungen und Erläuterungen zur Integrationsmutagenese findet man beispielsweise bei Schwarzer und Pühler (Bio/Technology 9,84-87 (1991)) oder Peters-Wendisch et al. (Microbiology 144, 915-927 (1998)).

Beispiele für Aminosäure produzierenden Stämme coryneformer Bakterien mit ausgeschaltetem pck-Gen sind der Lysin produzierende Stamm MH20-22BΔpck und der Threoninproduzierende Stamm DM368-2Δpck.

Zusätzlich kann es für die Produktion von L-Aminosäuren vorteilhaft sein, zusätzlich zur Abschaltung, des pck-Gens eines oder mehrere Enzyme des jeweiligen Biosyntheseweges zu überexprimieren.

So kann beispielsweise für die Herstellung von L-Lysin
- gleichzeitig das für die Dihydrodipicolinat-Synthase kodierende dapA-Gen überexprimiert werden (EP-B 0 197 335), oder
- gleichzeitig ein S-(2-Aminoethyl)-Cystein-Resistenz vermittelndes DNA-Fragment amplifiziert werden (EP-A 0 088 166).

So können beispielsweise für die Herstellung von L-Threonin
- gleichzeitig das für die Homoserin-Dehydrogenase kodierende hom-Gen (Peoples et al., Molecular Microbiology 2, 63-72 (1988)) oder die für eine "feed back resistente" Homoserin-Dehydrogenase kodierenden hom^{dr}- bzw- hom_{FBR} Allele (Archer et al., Gene 107, 53-59 (1991); Reinscheid et al., Journal of Bacteriology 173, 3228-3230 (1991)) überexprimiert werden.

Weiterhin kann es für die Produktion von L-Aminosäuren insbesondere Lysin und Threonin vorteilhaft sein, neben der Abschaltung des pck-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren insbesondere L-Lysin und L-Threonin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum der gewünschten L-Aminosäure gebildet hat. Dieses Ziel wird normalerweise innerhalb von 10 Stunden bis 160 Stunden erreicht.

Folgender Mikroorganismus wurde bei der Deutschen Sammlung für Mikrorganismen und Zellkulturen (DSMZ, Braunschweig, Deutschland) gemäß Budapester Vertrag hinterlegt:
- Escherichia coli Stamm DH5α/pK19mobsacBΔpck als DSM 13047

Das erfindungsgemäße Verfahren dient zur fermentativen Herstellung von L-Aminosäuren, insbesondere L-Asparaginsäure, L-Asparagin, L-Homoserin, L-Threonin, L-Isoleucin und L-Methionin mit coryneformen Bakterien, insbesondere der Herstellung von L-Lysin und L-Threonin.

### Beispiele

Die vorliegende Erfindung wird im folgenden anhand von Ausführungsbeispielen näher erläutert.

Zu diesem Zweck wurden unter anderem Versuche mit dem Lysin-Produzenten Corynebacterium glutamicum Stamm MH20-22B und dem Threonin-Produzenten Brevibacterium flavum Stamm DM368-2 durchgeführt. Stamm MH20-22B ist als DSM5715 (EP-B-0435 132) und Stamm DM368-2 als DSM5399 (EP-B- 0385 940) bei der Deutschen Sammlung für Mikroorganismen und Zellkulturen (DSMZ, Braunschweig, Deutschland) gemäß Budapester Vertrag hinterlegt.

### Beispiel 1

### Isolierung des pck-Gens

Zur Isolierung des PEP-Carboxykinase-Gens (pck) aus C. glutamicum wurde basierend auf dem Cosmid pHC79 (Hohn und Collins, Gene 11 (1980) 291-298) eine Cosmid-Genbank nach bekannter Methodik (Sambrook et al., Molecular Cloning, A Laboratory Handbook, 1989, Cold Spring Harbour Laboratory Press) angelegt. Dazu wurde aus C. glutamicum ATCC13032 chromosomale DNS isoliert (Eikmanns et al., Microbiology 140 (1994) 1817-1828) und mit dem Restriktionsenzym Sau3A partiell verdaut. Nach Ligation der erhaltenen Fragmente in die BamHI-Schnittstelle des Cosmids pHC79 wurde der Ansatz in die Proteinhülle des Bakteriophagen Lambda verpackt und der E. coli-Stamm ED8654 (Murray et al. Molecular and General Genetics 150 (1997) 53-61) damit transfiziert. Die Verpackung der rekombinanten Cosmide in die Proteinhülle des Phagen Lambda erfolgte nach einer Methode von Sternberg et al. (Gene 1 (1979) 255-280), die Transfektion von E. coli ED8654 nach einer Methode von Sambrook et al. (Molecular Cloning, A Laboratory Handbook, 1989, Cold Spring Harbour Laboratory Press). Aus insgesamt 30 der erhaltenen rekombinanten E. coli-Klone wurden die entsprechenden Cosmide isoliert (Sambrock et al., Molecular Cloning, A Laboratory Handbook, 1989, Cold Spring Harbour Laboratory Press) und einer Restriktionsanalyse mit dem Enzym HindIII unterzogen. Es zeigte sich, daß 24 der untersuchten Cosmide Inserts besaßen, und daß die Inserts Größen von ungefähr 35 kb aufwiesen. Insgesamt 2200 Cosmidtragende E. coli-Klone wurden vereinigt und aus diesem Gemisch nach bekanntem Verfahren (Sambrock et al., Molecular Cloning, A Laboratory Handbook, 1989, Cold Spring Harbour Laboratory Press) die Cosmid-DNA präpariert.

Zur Isolierung des pck-Gens aus C. glutamicum wurde die Cosmid-Genbank in die PEP-Carboxykinase-defekte E. coli-Mutante HG4 (Goldie and Sanwal, Journal of Bacteriology 141 (1980) 115-1121) nach bekanntem Verfahren (Sambrock et al., Molecular Cloning, A Laboratory Handbook, 1989, Cold Spring Harbour Laboratory Press) transformiert. Die Mutante HG4 ist aufgrund ihres PEP-Carboxykinase-Defektes nicht mehr in der Lage, auf Succinat als einziger Kohlenstoffquelle zu wachsen. Nach Transformation der Cosmid-Genbank in diese Mutante wurden insgesamt 1200 Klone erhalten. Von diesen zeigten insgesamt zwei Klone Wachstum auf M9-Minimalmedium (Sambrock et al., Molecular Cloning, A Laboratory Handbook, 1989, Cold Spring Harbour Laboratory Press) mit Succinat (0.4%) als einziger Kohlenstoffquelle. Nach Isolierung der entsprechenden Cosmide (Sambrock et al., Molecular Cloning, A Laboratory Handbook, 1989, Cold Spring Harbour Laboratory Press) aus diesen Klonen und erneuter Transformation in die E. coli-Mutante HG4 waren die resultierenden Klone erneut in der Lage, auf M9-Medium mit Succinat als einziger Kohlenstoffquelle zu wachsen.

Um das pck-Gen aus C. glutamicum auf einem kleineren Fragment einzugrenzen, wurden die zwei komplementierenden Cosmide mit den Restriktionsenzymen XhoI, ScaI und PvuII verdaut und nach bekannter Methode (Sambrock et al., Molecular Cloning, A Laboratory Handbook, 1989, Cold Spring Harbour Laboratory Press) auf einem 0,8%igen Agarosegel im elektrischen Feld aufgetrennt. Fragmente im Größenbereich über 3,0 kb wurden durch Elektroelution (Sambrock et al., Molecular Cloning, A Laboratory Handbook, 1989, Cold Spring Harbour Laboratory Press) aus dem Gel isoliert und in die SalI (XhoI-Verdau), bzw. in die Klenow-behandelte EcoRI-Schnittstelle (ScaI- und PvuII-Verdau) des Vektors pEK0 (Eikmanns et al., Gene 102 (1991) 93-98) ligiert. Mit den Ligationsansätzen wurde E. coli HG4 transformiert und die erhaltenen Transformanten erneut auf ihre Fähigkeit untersucht, auf Succinat als alleiniger Kohlenstoffquelle zu wachsen. In dem Transformationsansatz mit dem PvuII-Ligationsansatz zeigten sich sieben Klone, deren Plasmide der Mutante HG4 Wachstum auf Succinat erlaubten. Aus den rekombinanten Stämmen wurden die entsprechenden Plasmide isoliert und einer Restriktionskartierung unterzogen. Es zeigte sich, daß alle sieben Plasmide das gleiche 4.3-kb PvuII-Insert trugen, drei in der einen Orientierung, vier in der anderen. In Abhängigkeit von der Orientierung des Inserts im Vektor wurden die neu konstruierten Plasmide als pEK-pckA und pEK-pckB bezeichnet. Die Restriktionskarten beider Plasmide sind in Figur 1 und 2 dargestellt.

### Beispiel 2

### Sequenzierung des pck-Strukturgens und angrenzender Bereiche

Für die Sequenzierung wurde das circa 3.9 kb große EcoRI-Fragment aus pEK-pckA (dabei stammt eine EcoRI-Schnittstelle aus dem Vektor pEK0) nach bekannter Methode isoliert. Die überhängenden Enden des Fragmentes wurden mit Klenow-Polymerase zu glatten Enden aufgefüllt (Sambrock et al., Molecular Cloning, A Laboratory Handbook, 1989, Cold Spring Harbour Laboratory Press) und in die EcoRV-Schnittstelle des Vektors pGEM-5Zf(+) (Promega Corporation, Madison, WI, USA) ligiert. Die Insertion des so erzeugten Plasmides wurde durch die Kettenabbruch-Sequenziermethode (Sanger et al., Proceedings of the National Academy of Sciences USA, 74 (1977) 5463-5467) sequenziert. Sie ist als SEQ ID No. 1 dargestellt. Die erhaltene Nukleotidsequenz von 3935 bp wurde mit dem Programmpaket HUSAR (Release 3.0) des Deutschen Krebsforschungszentrums (DKFZ, Heidelberg, Deutschland) analysiert. Die Sequenzanalyse der Fragmente ergab ein offenes Leseraster von 1830 bp Länge, das für eine Protein bestehend aus 610 Aminosäuren kodiert.

### Beispiel 3

### Überexpression des pck-Gens

Durch Elektroporation mit nachfolgender Selektion auf Kanamycin (50 µg/ml) enthaltenden BHI-Agarplatten (Liebl et al., FEMS Microbiology Letters 65 (1989) 299-304) wurden die Plasmide pEK-pckA und pEK-pckB in den C. glutamicum Stamm ATCC13032 eingeführt und die resultierenden Stämme als ATCC13032/pEK-pckA und ATCC13032/pEK-pckB bezeichnet. Diese beiden Stämme und der Ausgangsstamm wurden in Luria-Bertani-Komplexmedium [Sambrook et al., Molecular Cloning, A laboratory manual (1989) Cold Spring Harbour Laboratory Press] gezüchtet und der PEP-Carboxykinase-Test entsprechend der Methode wie sie von Bentle and Lardy [Journal of Biological Chemistry 251 (1976) 2916-2921] beschrieben wurde, durchgeführt. Das Ergebnis der Analyse ist in Tabelle 1 dargestellt und zeigt, daß die PEP-Carboxykinase-Aktivität in den beiden Stämmen mit den Plasmiden pEK-pckA bzw. pEK-pckB 10- bis 12-fach höher ist als im Ausgangsstamm.

**Tabelle 1:**

| PEP-Carboxykinase-Aktivität in verschiedenen Stämmen | |
|---|---|
| Stamm | PEP-Carboxykinase (nmol min⁻¹ mg Protein⁻¹) |
| ATCC13032 | 120 |
| ATCC13032/pEK-pckA | 1270 |
| ATCC13032/pEK-pckB | 1510 |

### Beispiel 4

### Herstellung eines Integrationsplasmides für die Deletionsmutagenese des pck-Gens

Für die Inaktivierung des PEP-Carboxykinase-Gens wurde aus dem Vektor pEK-pckB (Figur 2) das EcoRI-SacI Fragment des pck-Gens isoliert und in den Vector pGEM-7Zf(+) (Promega Corporation, Madison, WI, USA) einligiert. Aus dem resultierenden Plasmid wurde ein pck-internes 1,07 kb HindII-HindIII-Fragment deletiert, anschließend das pck-Gen mit der 1,07-kb-Deletion als BfrI-SacI-Fragment isoliert und nach Auffüllen der überhängenden Enden in den in C. glutamicum nicht-replikativen Vektor pk19mobsacB (Schäfer et al., Gene 145, 69-73 (1994)) ligiert. In dem so konstruierten Integrationsplasmid pK19mobsacBΔpck (Figur 3) grenzt der 5'-Bereich des pck-Gens (350 bp) direkt an den 3'-Bereich des pck-Gens (340 bp); im Genom sind die beiden Bereiche durch 1071 bp voneinander getrennt. Bis zu diesem Schritt wurden alle Klonierungen in E. coli DH5α als Wirt durchgeführt.

### Beispiel 5

### Deletionsmutagenese des pck-Gens in dem Lysin-Produzenten MH20-22B.

Mit dem Integrationsplasmid pK19mobsacBΔpck wurde dann E. coli S17-1 transformiert (Simon et al., Bio/Technology 1,784-791 (1983)). Dieser Stamm ermöglicht den Transfer eines Plasmides nach Corynebacterium glutamicum durch Konjugation (Schäfer et al., Journal of Bacteriology 172 (1990) 1663-1666). Als Rezipient der Konjugation wurde der Lysinproduktionsstamm C. glutamicum MH20-22B verwendet (Schrumpf et al., Applied Microbiology and Biotechnology 37 (1992) 566-571)). Aus der Konjugation zwischen E. coli S17-1/pk19mobsacBΔpck und C. glutamicum MH20-22B und nachfolgenden Selektion auf Luria-Bertani-Agar-Platten mit Kanamycin (25 µg/ml) und Nalidixinsäure (50 µg/ml) wurden mehrere Transkonjuganten erhalten. Zur Selektion auf das zweite Rekombinationsereignis, das zur Excision des Vektors samt pck-Gen führen soll, wurden diese Transkonjuganten auf Antibiotika-freiem Luria-Bertani-Komplexmedium [Sambrook et al; Molecular Cloning, A laboratory manual (1989) Cold Spring Harbour Laboratory Press] mit 1% Glucose kultiviert und dann auf dem gleichen Medium plus 10% Saccharose plattiert. Das auf dem Vektor pk19mobsacB vorhandene sacB-Gen kodiert für das Enzym Levansucrase und führt zur Synthese von Levan aus Saccharose. Da Levan für C. glutamicum toxisch ist, können nur C. glutamicum Zellen, die das Integrationsplasmid verloren haben, auf Saccharosehaltigem Medium wachsen (Jäger et al., Journal of Bacteriology 174 (1992) 5462-5466). 30 Saccharoseresistente Klone wurden auf ihre Kanamycin-Sensitivität hin überprüft. Für 11 der getesteten Klone konnte neben der Saccharose-Resistenz auch die gewünschte Kanamycin-Sensitivität bestätigt werden. In diesen 11 Klonen war also der Vektorhintergrund wieder excisiert. Ob auch die gewünschte Deletion erfolgt war, wurde durch Analyse mittels Polymerase-Kettenreaktion (PCR) geprüft. Dafür wurde chromosomale DNA von einer Kolonie des Ausgangsstamms und von Kolonien der 11 Kanamycin-sensitiven Klone freigesetzt. Hierzu wurde die jeweilige Kolonie mit einem Zahnstocher von der Agarplatte abgenommen, in 50 µl H₂O suspendiert und 5 Minuten bei 95°C inkubiert. Jeweils 1 µl der erhaltenden Lösung wurden als Template in die PCR eingesetzt. Als Primer wurden Oligonukleotide verwendet, die die Bereiche von Nukleotid 2136 bis 2158 und von 3815 bis 3793 in der SEQ ID No. 1 abdecken. Die PCR-Bedingungen waren: Vorabdenaturierung: 150 Sekunden bei 94°C; Denaturierung 60 Sekunden bei 94°C; Hybridisierung 30 Sekunden bei 60°C; Amplifizierung 120 Sekunden bei 72°C; 30 Zyklen, End-Extension 240 Sekunden bei 72°C. Im Ansatz mit der DNA des Ausgangsstammes wurde aufgrund der gewählten Primer ein PCR-Produkt von 1,68 kb erwartet. In der PCR mit der pck-Deletionsmutante wurde ein PCR-Produkt von 0,61 kb erwartet. Bei einem Klon wurde ein 0,61 kb großes PCR-Produkt erhalten. Dadurch wurde die gewünschte Deletion des internen 1071 bp großen pck-Fragmentes bei diesem Klon nachgewiesen. Der Klon wurde als MH20-22BΔpck bezeichnet. In den Ansätzen der übrigen Klonen wurde das 1,68 kb PCR-Produkt nachgewiesen. In diesen war der Vektor also so excisiert, daß die genomische Ausgangssituation wieder hergestellt war.

Der Stamm MH20-22BΔpck und der Ausgangsstamm MH20-22B wurden in Luria-Bertani-Komplexmedium plus 1% Glucose angezogen und der PEP-Carboxykinase-Test wurde entsprechend der Methode, wie sie bei Bentle and Lardy (Journal of Biological Chemistry 251 (1976) 2916-2921) beschrieben ist, durchgeführt. Das Ergebnis der Analyse (Tabelle 2) zeigt, daß in der Mutante MH20-22BΔpck im Gegensatz zum Ausgangsstamm MH20-22B keine PEP-Carboxykinase-Aktivität mehr nachweisbar ist.

**Tabelle 2:**

| PEP-Carboxykinase-Aktivität in verschiedenen Stämmen | |
|---|---|
| Stamm | *PEP-Carboxykinase (nmol min⁻¹ mg Protein⁻¹) |
| MH20-22B | 65 |
| MH20-22BΔpck | < 3 * |

| | |
|---|---|
| * 3 nmol min⁻¹ mg Protein⁻¹ ist die Nachweisgrenze | |

### Beispiel 6

### Produktion von L-Lysin

Zur Untersuchung der Auswirkung der Inaktivierung des PEP-Carboxykinase-Gens auf die Lysinproduktion wurde der Stamm MH20-22B (Schrumpf et al., Applied Microbiology and Biotechnology 1992, 37:566-571) sowie die PEP-Carboxykinase-negative Mutante MH20-22BΔpck (Beispiel 5) in Luria-Bertani-Komplexmedium plus 1% Glucose kultiviert und das Fermentationsmedium CGXII (Keilhauer et al., Journal of Bacteriology 1993, 175:5595-5603) aus den beiden Vorkulturen beimpft (5% Inokulum, Optische Dichte bei 600 nm circa 0,5). Das Medium enthielt zusätzlich 3 mM Leucin, da die beiden Stämme Leucin-auxotroph sind. Die Ansätze bestanden aus jeweils 60 ml Kultur, die in 500 ml-Erlenmeyer-Kolben mit Schikanen enthalten waren. Nach Kultivierung für 24 Stunden bei 28°C auf einem Rotationsschüttler vom Typ Certomat S/50 (Firma B. Braun Biotech International, Melsungen, Deutschland) bei 120 Upm wurde die Konzentration des in das Medium ausgeschiedenen Lysins bestimmt.

Die Bestimmung der Aminosäurekonzentration erfolgte mittels Hochdruckflüssigkeitschromatographie (Jones und Gilligan, Journal of Chromatography 1983, 266:471-482). Das Ergebnis der Fermentation ist in Tabelle 3 dargestellt.

**Tabelle 3:**

| Lysinkonzentration im Kulturüberstand der Stämme MH20-22B und MH20-22BΔpck | |
|---|---|
| Stamm | L-Lysin (mM) |
| MH20-22B | 54 |
| MH20-22BΔpck | 65 |

### Beispiel 7

### Deletionsmutagenese des pck-Gens in dem Threonin-Produzenten DM368-2

Mit dem E. coli-Stamm S17-1/ pk19mobsacBΔpck wurde wie im Fall des Lysinproduzenten MH20-22B eine Konjugation mit dem Threoninproduzenten DM368-2 mit anschließender Selektion auf die erste und zweite Rekombination durchgeführt (siehe Beispiel 5). Von 30 Saccharose-resistenten Klonen waren 14 Kanamycin-sensitiv. Von diesen konnte in zweien, als DM368-2Δpck16 und DM368-2Δpck18 bezeichneten Klonen mit Hilfe der in Beispiel 5 beschriebenen PCR-Analyse die 1071 bp-Deletion im pck-Gen nachgewiesen werden.

Ein Enzymtest mit dem Ausgangsstamm DM368-2 und den beiden pck-Deletionsstämmen DM368-2Δpck16 und DM368-2Apck18, durchgeführt wie in Beispiel 5 beschrieben, zeigte, daß in diesen Mutanten keine PEP-Carboxykinase-Aktivität nachweisbar ist (Tabelle 4).

**Tabelle 4**

| PEP-Carboxykinase-Aktivität in verschiedenen Stämmen | |
|---|---|
| Stamm | PEP-Carboxykinase (nmol min⁻¹ mg Protein⁻¹) |
| DM368-2 | 79 |
| DM368-2BΔpck16 | < 3 * |
| DM368-2BΔpck18 | < 3 * |

| | |
|---|---|
| *3 nmol min⁻¹ mg Protein⁻¹ ist die Nachweisgrenze | |

### Beispiel 8

### Produktion von L-Threonin

Analog zu den Experimenten zur L-Lysinproduktion wurde auch die Akkumulation von Threonin im Kulturüberstand des PEP-Carboxykinase-defekten Stammes DM368-2BΔpck16 im Vergleich mit dem Ausgangsstamm DM368-2 untersucht. Dazu wurden die beiden Stämme in Luria-Bertani-Komplexmedium plus 1% Glucose gezüchtet und das Fermentationsmedium CGXII aus den Vorkulturen beimpft. Nach Kultivierung für 24 Stunden bei 28°C auf dem Rotationsschüttler bei 120 Upm wurde die Konzentration des in das Medium ausgeschiedenen Threonins bestimmt.

Die Bestimmung der Aminosäurekonzentration erfolgte mittels Hochdruckflüssigkeitschromatographie (siehe oben). Das Ergebnis der Fermentation ist in Tabelle 5 dargestellt.

**Tabelle 5:**

| Threoninkonzentration im Kulturüberstand der Stämme DM 368-2 und DM,368-2Δpck16 | |
|---|---|
| Stamm | L-Threonin (mM) |
| DM368-2 | 8 |
| DM368-2Δpck16 | 22 |

### Abbildungen

Folgende Figuren sind beigefügt:
- Figur 1: Restriktionskarte des Plasmides pEK-pckA
- Figur 2: Restriktionskarte des Plasmides pEK-pckB
- Figur 3: Restriktionskarte des Plasmides pk19mobsacBΔpck

Bei der Angabe der Basenpaarzahlen handelt es sich um ca.-Werte, die im Rahmen der Reproduzierbarkeit erhalten werden.

Die verwendeten Abkürzungen und Bezeichnungen haben folgende Bedeutung:
- sacB:: sacB-Gen
- ori V:: Replikationsursprung V
- ori T:: Replikationsursprung für den Transfer
- Km-r:: Kanamycin Resistenz
- KpnI:: Schnittstelle des Restriktionsenzyms KpnI
- HindIII:: Schnittstelle des Restriktionsenzyms HindIII
- HindII:: Schnittstelle des Restriktionsenzyms HindII
- PstI:: Schnittstelle des Restriktionsenzyms PstI
- SphI:: Schnittstelle des Restriktionsenzyms SphI
- XbaI:: Schnittstelle des Restriktionsenzyms XbaI
- SalI:: Schnittstelle des Restriktionsenzyms SalI
- SacI:: Schnittstelle des Restriktionsenzyms SacI
- BfrI:: Schnittstelle des Restriktionsenzyms BfrI
- ScaI:: Schnittstelle des Restriktionsenzyms ScaI
- BamHI:: Schnittstelle des Restriktionsenzyms BamHI
- EcoRI:: Schnittstelle des Restriktionsenzyms EcoRI
- pck*'*:: 3'-terminales Fragment des pck-Gens
- pck*''*:: 5'-terminales Fragment des pck-Gens
- pck:: pck-Gen

## Patentansprüche

1. Isoliertes coryneformes Bakterium, bei dem die Expression eine Polynukleotids ausgeschaltet ist, das für ein Polypeptid kodiert, das eine Aminosäuresequenz hat, die mindestens 90 % identisch ist mit der Aminosäuresequenz von SEQ ID NO:2 , wobei das Polypeptid die Funktion einer Phosphoenolpyruvat-Carboxykinase hat.

2. Bakterium gemäss Anspruch 1, wobei das genannte Bakterium zur Gattung Corynebacterium gehört.

3. Bakterium gemäss Anspruch 2, wobei das genannte Bakterium zur Spezies Corynebacterium glutamicum gehört.

4. Bakterium gemäss den Ansprüchen 1, 2 oder 3, wobei das Polynukleotid ausgewählt ist aus der Gruppe:
a) der in SEQ ID NO:1 wiedergegebenen Nukleotidsequenz oder
b) Nukleotidsequenzen, die der Sequenz SEQ ID NO:1 innerhalb der Degeneration des genetischen Codes entsprechen, oder
c) Nukleotidsequenzen mit neutralen Sinnmutationen in SEQ ID NO:1.

5. Bakterium gemäss Anspruch 1, wobei das genannte Polypeptid die in SEQ ID NO:2 wiedergegebene Aminosäuresequenz hat.

6. Bakterium gemäß einem oder mehreren der Ansprüche 1 bis 5, wobei die Ausschaltung durch Insertion oder Deletion von mindestens einem Basenpaar in dem genannten Polynukleotid erzielt wird.

7. Vektor pkl9mobsacBäpck, hinterlegt in dem Stamm E. coli DH5α unter der Nummer DSM 13047

8. Als Wirtszelle dienendes coryneformes Bakterium, das den Vektor gemäss Anspruch 7 enthält oder in das die Δpck-Deletion eingebaut wurde.

9. Verfahren zur Herstellung von L-Aminosäuren, umfassend das Fermentieren des genannten aminosäureproduzierenden Bakteriums nach einem der Ansprüche 1 bis 6 oder 8 in einem Medium.

10. Verfahren zur Herstellung von L-Aminosäuren, umfassend das Fermentieren coryneformer Bakterien, in denen die intrazelluläre Aktivität des Polypeptids mit der in SEQ ID NO:2 wiedergegebenen Aminosäuresequenz ausgeschaltet ist, insbesondere die Expression der Polynukleotide mit den Sequenzen ausgeschaltet ist, ausgewählt aus der Gruppe:
a) der in SEQ ID NO:1 dargelegten Nukleotisequenz oder
b) Nukleotidsequenzen, die der SEQ ID NO: 1 innerhalb der Degeneration des genetischen Codes entsprechen, oder
c) Nukleotidsequenzen mit neutralen Sinnmutationen in SEQ ID NO:1,
die für ein Polypeptid mit der Funktion einer Phosphoenolpyruvat-Carboxykinase kodieren.

11. Verfahren gemäss den Ansprüchen 9 oder 10, umfassend
a) Fermentation der genannten coryneformen Bakterien, die die gewünschte L-Aminosäure produzieren,
b) Konzentration der genannten L-Aminosäure in dem Medium oder in den Zellen der Bakterien und
c) Isolieren der L-Aminosäure.

12. Verfahren gemäss den Ansprüchen 9 bis 11,
bei dem man Bakterien der Gattung Corynebacterium einsetzt.

13. Verfahren gemäss Anspruchen 12,
bei dem man Bakterien der Spezies Corynebacterium glutamicum einsetzt.

14. Verfahren gemäss den Ansprüchen 9 bis 13, bei dem man die Ausschaltung des pck-Gens durch Integrationsmutagenese mit Hilfe des Plasmids pk19mobsacBΔpck, hinterlegt in DSM 13047, erzielt.

15. Verfahren gemäss den Ansprüchen 9 bis 14,
bei dem man für die Herstellung von L-Lysin Bakterien fermentiert, in denen man
• gleichzeitig das für das Dihydrodipicolinat-Synthase kodierende dapA-Gen überexprimiert, und/oder
• gleichzeitig ein S-(2-Aminoethyl)-Cystein-Resistenz vermittelndes DNA-Fragment amplifiziert.

16. Verfahren gemäß den Ansprüchen 9 bis 14,
bei dem man für die Herstellung von L-Threonin Bakterien fermentiert, in denen man gleichzeitig das für die Homoserin-Dehydrogenase kodierende hom-Gen und/oder für eine "feed back resistente" Homoserin-Dehydrogenase kodierende hom^{dr}-bzw. hom^{FBR}-Allele überexprimiert.

## Claims

1. Isolated coryneform bacterium in which expression of a polynucleotide coding for a polypeptide having an amino acid sequence which is at least 90% identical to the amino acid sequence of SEQ ID NO: 2 has been switched off, wherein the polypeptide has the function of a phosphoenolpyruvate carboxykinase.

2. Bacterium according to Claim 1, which belongs to the genus Corynebacterium.

3. Bacterium according to Claim 2, which belongs to the species Corynebacterium glutamicum.

4. Bacterium according to Claim 1, 2 or 3, wherein the polynucleotide is selected from the group:
a) the nucleotide sequence depicted in SEQ ID NO: 1 or
b) nucleotide sequences corresponding to the sequence SEQ ID NO: 1 within the degeneracy of the genetic code, or
c) nucleotide sequences having neutral sense mutations in SEQ ID NO: 1.

5. Bacterium according to Claim 1, wherein the said polypeptide has the amino acid sequence depicted in SEQ ID NO: 2.

6. Bacterium according to one or more of Claims 1 to 5, wherein the switching-off is achieved by inserting or deleting at least one base pair in the said polynucleotide.

7. Vector pk19mobsacBΔpck, deposited in E. coli strain DH5α under DSM 13047.

8. Coryneform bacterium serving as a host cell, which comprises the vector according to Claim 7 or into which the Δpck deletion has been incorporated.

9. Process for producing L-amino acids, comprising fermenting the said amino acid-producing bacterium according to any of Claims 1 to 6 or 8 in a medium.

10. Process for producing L-amino acids, comprising fermenting coryneform bacteria in which the intracellular activity of the polypeptide having the amino acid sequence depicted in SEQ ID NO: 2, in particular expression of the polynucleotides having the sequences selected from the group:
a) the nucleotide sequence set forth in SEQ ID NO: 1 or
b) nucleotide sequences corresponding to SEQ ID NO: 1 within the degeneracy of the genetic code, or
c) nucleotide sequences having neutral sense mutations in SEQ ID NO: 1,
which code for a polypeptide having the function of a phosphoenolpyruvate carboxykinase, has been switched off.

11. Process according to Claim 9 or 10, comprising
a) fermenting the said coryneform bacteria which produce the desired L-amino acid,
b) concentrating the said L-amino acid in the medium or in the cells of the bacteria, and
c) isolating the L-amino acid.

12. Process according to Claims 9 to 11, in which bacteria of the genus Corynebacterium are employed.

13. Process according to Claim 12, in which bacteria of the species Corynebacterium glutamicum are employed.

14. Process according to Claims 9 to 13, in which the pck gene is switched off by integration mutagenesis with the aid of plasmid pkl9mobsacBΔpck, deposited in DSM 13047.

15. Process according to Claims 9 to 14, in which L-lysine is produced by fermenting bacteria in which
• the dapA gene coding for dihydrodipicolinate synthase is overexpressed at the same time, and/or
• a DNA fragment imparting S-(2-aminoethyl)-cysteine resistance is amplified at the same time.

16. Process according to Claims 9 to 14, in which L-threonine is produced by fermenting bacteria in which the hom gene coding for homoserine dehydrogenase and/or hom^{dr} and, respectively, hom^{FBR} alleles coding for a feed back-resistant homoserine dehydrogenase are overexpressed at the same time.

## Revendications

1. Bactérie coryneforme isolée, dans laquelle est éteinte l'expression d'un polynucléotide, qui code pour un polypeptide, qui a une séquence d'acides aminés qui est identique au moins à 90% à la séquence d'acides aminés SEQ ID n° 2, le polypeptide ayant la fonction d'une phosphoénolpyruvate-carboxykinase.

2. Bactérie selon la revendication 1, dans laquelle la bactérie citée appartient à la souche Corynebacterium.

3. Bactérie selon la revendication 2, dans laquelle la bactérie citée appartient à la souche Corynebacterium glutamicum.

4. Bactérie selon la revendication 1, 2 ou 3, dans laquelle le polynucléotide est choisi dans le groupe :
a) de la séquence nucléotidique reproduite dans la SEQ ID n° 1, ou
b) de séquences nucléotidiques qui correspondent à la séquence SEQ ID n° 1 dans la dégénération du code génétique, ou
c) des séquences nucléotidiques avec des mutations de sens neutres dans la SEQ ID n° 1.

5. Bactérie selon la revendication 1, dans laquelle le polypeptide cité a la séquence d'acides aminés reproduite dans la SEQ ID n° 2.

6. Bactérie selon une ou plusieurs des revendications 1 à 5, dans laquelle on obtient l'extinction par insertion ou délétion d'au moins une paire de bases dans le polynucléotide cité.

7. Vecteur pk19mobsacBΔpck, déposé dans la lignée E. coli DH5α sous le numéro DSM 13047.

8. Bactérie coryneforme servant de cellule hôte qui contient le vecteur selon la revendication 7 ou à laquelle la délétion Δpck a été intégrée.

9. Procédé de fabrication d'acides L-aminés comprenant la fermentation de la bactérie citée reproductrice d'acides aminés selon l'une quelconque des revendications 1 à 6 ou 8 dans un milieu.

10. Procédé de fabrication d'acides L-aminés comprenant la fermentation de bactéries coryneformes, dans lequel l'activité intracellulaire du polypeptide avec la séquence d'acides aminés reproduite dans la SEQ ID n° 2 est éteinte, en particulier l'expression des polynucléotides avec les séquences choisies dans le groupe :
a) de la séquence nucléotidique représentée dans la SEQ ID n° 1, ou
b) de séquences nucléotidiques qui correspondent à la SEQ ID n° 1 dans la dégénération du code génétique, ou
c) de séquences nucléotidiques avec des mutations de sens neutres dans la SEQ ID n° 1,
qui codent pour un polypeptide avec la fonction d'une phosphoénolpyruvate-carboxykinase.

11. Procédé selon la revendication 9 ou 10, comprenant
a) la fermentation des bactéries coryneformes citées qui produisent l'acide L-aminé souhaité,
b) la concentration de l'acide L-aminé cité dans le milieu ou dans les cellules des bactéries, et
c) l'isolement de l'acide L-aminé.

12. Procédé selon les revendications 9 à 11, dans lequel on utilise des bactéries de la souche Corynebacterium.

13. Procédé selon la revendication 12, dans lequel on utilise des bactéries de la souche Corynebacterium glutamicum.

14. Procédé selon les revendications 9 à 13, dans lequel on obtient l'extinction du gène pck par mutagenèse d'intégration à l'aide du plasmide pk19mobsacBΔpck, déposé sous le numéro DSM 13047.

15. Procédé selon les revendications 9 à 14, dans lequel, pour la fabrication de L-lysine, on fait fermenter des bactéries, dans lesquelles :
• on surexprime simultanément le gène dapA codant pour la dihydrodipicolinate-synthase, et/ou
• on amplifie simultanément un fragment d'ADN transmettant une résistance à la S-(2-aminoéthyl)-cystéine.

16. Procédé selon les revendications 9 à 14, dans lequel on fait fermenter des bactéries pour la fabrication de L-thréonine, dans lesquelles on surexprime simultanément le gène hom codant pour l'homosérine-déshydrogénase et/ou des allèles hom^{dr} ou hom^{FBR} codant pour une homosérine-déshydrogénase "résistant à la rétroaction".
